# EUROPEAN PATENT SPECIFICATION

(11) **EP 1 095 654 B1**
(45) Date of publication and mention of the grant of the patent: **19.11.2003**
(21) Application number: 01200197.0
(22) Date of filing: 19.12.1994
(51) Int. Cl.: A61K 31/445, A61K 31/40

(54) **Use of raloxifene and its analogs for the manufacture of a medicament for the Inhibition of LDL oxidation and atherosclerosis**
Verwendung von Raloxifen und dessen Analogen zur Herstellung eines Medikaments zur Hemmung der LDL Oxidation und der Atherosclerose
Utilisation du raloxifene et de ses analogues dans la fabrication d'un medicament pour l'Inhibition de l'oxidation de LDL et de l'athérosclérose

(30) Priority: 21.12.1993 US 170606
(43) Date of publication of application: 02.05.2001
(62) Divisional of application: 94309461.5
(73) Proprietor: ELI LILLY AND COMPANY, Indianapolis, Indiana 46285 (US)
(72) Inventor: Zuckerman, Steven Harold, Indianapolis, Indiana 46250 (US)
(74) Representative: Burnside, Ivan John

(56) References cited:
- EP-A- 0 605 193
- US-A- 3 983 245
- H.S. KEEPING ET AL.: "Modulation of rat uterine progesterone receptor levels and peroxidase activity by tamoxifen citrate, LY117018, and estradiol" ENDOCRINOLOGY, vol. 111, no. 6, pages 2046-2054, XP002163158
- DATABASE DIALOG FILE 445:R&D FOCUS [Online] IMSworld Publications Ltd.; Dialog Accession no. 00004016, XP002114929 & "Products nearing market with Lilly" R&D FOCUS DRUG NEWS, 6 September 1993 (1993-09-06),
- DATABASE FILE 187, FDC REPORTS [Online] Dialog Information Services; 1987-1993, Accession No. 00089403, XP002163160 & "Lilly's raloxifene entering phase III for osteoporosis" THE PINK SHEET, vol. 55, no. 16, 19 April 1993 (1993-04-19),

## Description

Atherosclerosis as manifested in its major clinical complication, ischaemic heart disease, continues to be a major cause of death in industrialized countries. It is now well accepted that atherosclerosis can begin with local injury to the arterial endothelium followed by proliferation of arterial smooth muscle cells from the medial layer to the intimal layer along with deposition of lipid and accumulation of macrophage derived foam cells in the lesion. As the atherosclerotic plaque develops it progressively includes more and more of the affected blood vessel and can eventually lead to ischaemia or infarction. Therefore, it is desirable to provide methods of inhibiting the progression of atherosclerosis in patients in need thereof.

There is evidence based on animal and laboratory findings that peroxidation of LDL lipid, such as the unsaturated fatty acid portions of LDL cholesteryl esters and phospholipids, facilitates the accumulation of cholesterol in monoctye/macrophages which eventually are transformed into foam cells and become deposited in the sub-endothelial space of the vessel wall. The accumulation of foam cells in the vessel wall is recognized as an early event in the formation of an atherosclerotic plaque. Thus, it is believed that peroxidation of LDL lipid is an important prerequisite to the facilitated accumulation of cholesterol in the vessel wall and the subsequent formation of an atherosclerotic plaque. For example, it has been shown that monocyte/macrophages take up and degrade native LDL at relatively low rates and without marked accumulation of cholesterol. In contrast, oxidized LDL is taken up by these monocyte/macrophages at much higher rates and with marked accumulation of cholesterol (Parthasarathy et *al., J. Clin Invest.* 77, 641 (1986)]. It is therefore desirable to provide methods of inhibiting LDL lipid peroxidation in a patient in need thereof.

It has been shown that 2,2'-bis(3,5-di-tertiary-butyl-4-hydroxyphenylthio)propane (also known as probucol), which is a known antioxidant, may prevent the progression of atherosclerosis in a manner which is independent of its effect on lowering plasma cholesterol levels [See Kita *et al. Proc. Natl. Acad. Sci.* USA 84, 5928 (1987); Carew *et al., Proc. Natl. Acad. Sci.* USA 84, 7725, (1987)]. It is believed that antioxidants, such as probucol, may prevent or inhibit the development of atherosclerosis by inhibiting the peroxidation of LDL and thus preventing the facilitated accumulation of cholesterol in monocyte/macrophages which eventually are transformed into foam cells and become deposited in the sub-endothelial space of the vessel wall [See Parthasarathy et *al. J. Clin. Invest.* 77, 641 (1986)]. Accordingly, it is desirable to provide a method of inhibiting the peroxidation of LDL.

The present invention relates to certain compounds which are useful as inhibitors of LDL lipid oxidation, atherosclerosis, advanced glycosylation end products (AGE) or glycation of AGE proteins, and superoxide anions and other reactive oxygen intermediates.

The invention provides the use of a compound of the formula 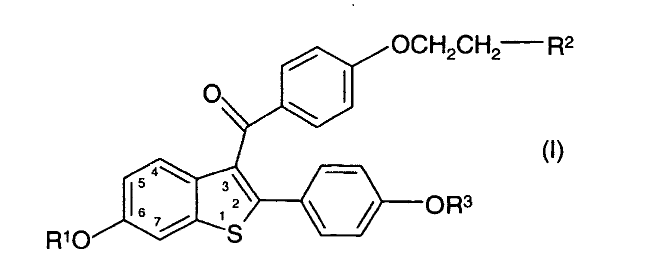 wherein R¹ and R³ are independently hydrogen, -CH₃, 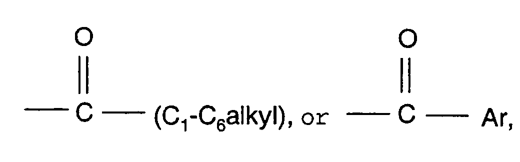 wherein Ar is optionally substituted phenyl;
R² is 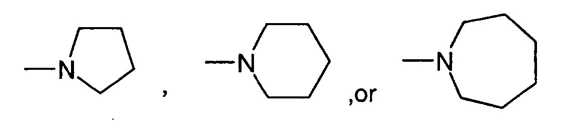 and
pharmaceutically acceptable salts and solvates thereof, in the manufacture of a medicament for the treatment of a disorder associated with superoxide anions and other reactive oxygen intermediates.

The current invention concerns the discovery that a select group of compounds, those of formula I, are useful for inhibiting LDL oxidation, atherosclerosis, AGE, and superoxide anions and other reactive oxygen intermediates. The uses provided by this invention are practiced by administering to a human or other mammal in need a dose of a compound of formula I or a pharmaceutically acceptable salt or solvate thereof, that is effective to inhibit the above. The term inhibit is defined to include its generally accepted meaning which includes phrophylactically treating a human subject to incurring one of the above and holding in check and/or treating an existing problem listed above. As such, the present uses include both medical therapeutic and/or prophylactic treatment, as appropriate.

Generally, the compound is formulated with common excipients, diluents or carriers, and compressed into tablets, or formulated as elixirs or solutions for convenient oral administration, or administered by the intramuscular or intravenous routes. The compounds can be administered transdermally, and may be formulated as sustained release dosage forms and the like.

The compounds of formula I used in the methods of the current invention can be made according to established procedures, such as those detailed in U.S. Patent Nos. 4,133,814, 4,418,068, and 4,380,635. In general, the process starts with a benzo[b]thiophene having a 6- hydroxyl group and a 2-(4-hydroxyphenyl) group. The starting compound is protected, acylated, and deprotected to form the formula I compounds. Examples of the preparation of such compounds are provided in the U.S. patents discussed above. Optionally substituted phenyl includes phenyl and phenyl substituted once or twice with C₁-C₆ alkyl, C₁-C₄ alkoxy, hydroxy, nitro, chloro, fluoro, or tr(chloro or fluoro)methyl.

Included in the invention is the use of the following compound, known as raloxifene:

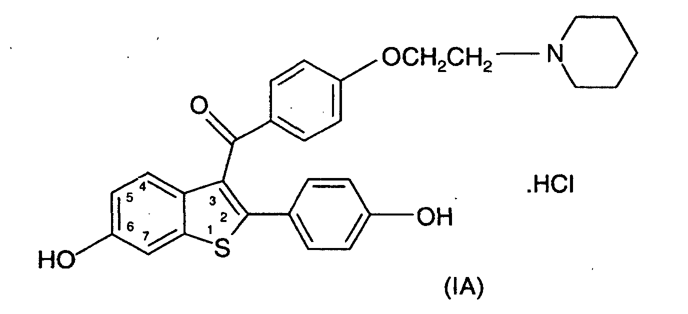

The compounds used in this invention form pharmaceutically acceptable acid and base addition salts with a wide variety of organic and inorganic acids and bases and include the physiologically acceptable salts which are often used in pharmaceutical chemistry. Such salts are also part of this invention. Typical inorganic acids used to form such salts include hydrochloric, hydrobromic, hydroiodic, nitric, sulfuric, phosphoric, hypophosphoric and the like. Salts derived from organic acids, such as aliphatic mono and dicarboxylic acids, phenyl substituted alkanoic acids, hydroxyalkanoic and hydroxyalkandioic acids, aromatic acids, aliphatic and aromatic sulfonic acids, may also be used. Such pharmaceutically acceptable salts thus include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, β-hydroxybutyrate, butyne-1,4-dioate, hexyne-1,4-dioate, caprate, caprylate, chloride, cinnamate, citrate, formate, fumarate, glycollate, heptanoate, hippurate, lactate, malate, maleate, hydroxymaleate, malonate, mandelate, mesylate, nicotinate, isonicotinate, nitrate, oxalate, phthalate, teraphthalate, phosphate, monohydrogenphosphate, dihydrogenphosphate, metaphosphate, pyrophosphate, propiolate, propionate, phenylpropionate, salicylate, sebacate, succinate, suberate, sulfate, bisulfate, pyrosulfate, sulfite, bisulfite, sulfonate, benzene-sulfonate, p-bromophenylsulfonate, chlorobenzenesulfonate, ethanesulfonate, 2-hydroxyethanesulfonate, methane-sulfonate, naphthalene-1-sulfonate, naphthalene-2-sulfonate, p-toluenesulfonate, Xylenesulfonate and tartarate. A preferable salt is the hydrochloride salt.

The pharmaceutically acceptable acid addition salts are typically formed by reacting a compound of formula I with an equimolar or excess amount of acid. The reactants are generally combined in a mutual solvent such as diethyl ether or benzene. The salt normally precipitates out of solution within about one hour to 10 days and can be isolated by filtration or the solvent can be stripped off by conventional means.

Bases commonly used for formation of salts include ammonium hydroxide and alkali and alkaline earth metal hydroxides, carbonates and bicarbonates, as well as aliphatic and aromatic amines, aliphatic diamines and hydroxy alkylamines. Bases especially useful in the preparation of addition salts include ammonium hydroxide, potassium carbonate, sodium bicarbonate, calcium hydroxide, methylamine, diethylamine, ethylene diamine, cyclohexylamine and ethanolamine.

The pharmaceutically acceptable salts generally have enhanced solubility characteristics compared to the compound from which they are derived, and thus are often more amenable to formulation as liquids or emulsions.

Pharmaceutical formulations can be prepared by procedures known in the art. For example, the compounds can be formulated with common excipients, diluents, or carriers, and formed into tablets, capsules, suspensions and powders. Examples of excipients, diluents, and carriers that are suitable for such formulations include the following: fillers and extenders such as starch, sugars, mannitol, and silicic derivatives; binding agents such as carboxymethyl cellulose and other cellulose derivatives, alginates, gelatin, and polyvinyl pyrrolidone; moisturizing agents such as glycerol; disintegrating agents such as agaragar, calcium carbonate, and sodium bicarbonate; agents for retarding dissolution such as paraffin; resorption accelerators such as quaternary ammonium compounds; surface active agents such as cetyl alcohol, glycerol monostearate; adsorptive carriers such as kaolin and bentonite; and lubricants such as talc, calcium and magnesium stearate, and solid polyethyl glycols.

The compounds can also be formulated as elixirs or solutions for convenient oral administration or as solutions appropriate for parenteral administration, for instance by intramuscular, subcutaneous or intravenous routes. Additionally, the compounds are well suited to formulation as sustained release dosage forms and the like. The formulations can be so constituted that they release the active ingredient only or preferably in a particular part of the intestinal tract, possibly over a period of time. The coatings, envelopes, and protective matrices may be made, for example, from polymeric substances or waxes.

Artherosclerosis is a disease state characterized by the development and growth of atherosclerotic lesions or plaque. The identification of those patients who are in need of treatment for atherosclerosis is well within the ability and knowledge of one skilled in the art. For example, individuals who are either suffering from clinically significant atherosclerosis or who are at risk of developing clinically significant atherosclerosis are patients in need of treatment for atherosclerosis. A clinician skilled in the art can readily determine, by the use of clinical tests, physical examination and medical/family history, if an individual is a patient in need of treatment for atherosclerosis.

An effective antiatherosclerotic amount of a compound of formula (1) is an amount which is effective in inhibiting development or growth of atherosclerosis in a patient in need thereof. As such, successful treatment of a patient for atherosclerosis is understood to include effectively slowing, interrupting, arresting, or stopping atherosclerotic lesion or plaque development or growth and does not necessarily indicate a total elimination of the artherosclerosis. It is further understood and appreciated by those skilled in the art that successful treatment for atherosclerosis can include prophylaxis in preventing atherosclerotic lesion or plaque formation.

The invention encompasses the inhibition of superoxide anions, and other reactive oxygen intermediates (ROI) levels/amounts. The compounds, due to their free radical scavenging abilities, are believed to be useful in such instances as inhibiting Acute Respiratory Distress Syndrome, the systemic inflammatory response often observed following cardiopulmonary bypass surgery, pancreatitis, and long term respiratory therapy problems associated with high oxygen levels.

Peroxidation of LDL lipid, such as the unsaturated fatty acid portions of LDL cholesteryl esters and phosholipids, is known to facilitate the deposition of cholesterol in macrophages which subsequently are deposited in the vessel wall and are transformed into foam cells. The identification of those patients who are in need of inhibition of peroxidation of LDL lipid is well within the ability and knowledge of one skilled in the art. For example, those individuals who are in need of treatment for atherosclerosis as defined hereinabove, are also patients who are in need of inhibition of peroxidation of LDL lipid. An effective antioxidant amount of a compound of formula (1) is an amount which is effective in inhibiting the peroxidation of LDL lipid in the patient's blood.

An effective dose of the compounds described for the complications listed can be determined by the use of conventional techniques and by observing results obtained under analogous circumstances. In determining the effective dose, a number of factors are considered including, but not limited to: the species of patient; its size, age and general health; the specific disease involved; the degree of or involvement or the severity of the disease; the response of the individual patient; the particular compound administered; the mode of administration; the bioavailability characteristics of the preparation administered; the dose regimen selected; and the use of concomitant medication.

Generally accepted and effective daily doses will be from 0.1 to 1000 mg/day, and more typically from 50 to 200 mg/day. Such dosages will be administered to a subject in need of treatment from once to about three times each day, or more often as needed to effectively inhibit one of the listed problems.

It is usually preferred to administer a compound of formula I in the form of an acid addition salt, as is customary in the administration of pharmaceuticals bearing a basic group, such as the piperidino ring. It is also advantageous to administer such a compound by the oral route. For such purposes the following oral dosage forms are available.

### Formulations

In the formulations which follow, "Active ingredient" means a compound of formula I.

### Formulation 1: Gelatin Capsules

Hard gelatin capsules are prepared using the following:

| **Ingredient** | **Quantity (mg/capsule)** |
|---|---|
| Active ingredient | 0.1 - 1000 |
| Starch, NF | 0 - 650 |
| Starch flowable powder | 0 - 650 |
| Silicone fluid 350 centistokes | 0 - 15 |

The ingredients are blended, passed through a 0.3mm (No. 45 mesh U.S.) sieve, and filled into hard gelatin capsules.

Examples of specific capsule formulations of the compound of formula 1 wherein the compound is raloxifene, include those shown below:

### Formulation 2: Raloxifene capsule

| **Ingredient** | **Quantity (mg/capsule)** |
|---|---|
| Raloxifene | 1 |
| Starch, NF | 112 |
| Starch flowable powder | 225.3 |
| Silicone fluid 350 centistokes | 1.7 |

### Formulation 3: Raloxifene capsule

| **Ingredient** | **Quantity (mg/capsule)** |
|---|---|
| Raloxifene | 5 |
| Starch, NF | 108 |
| Starch flowable powder | 225.3 |
| Silicone fluid 350 centistokes | 1.7 |

### Formulation 4: Raloxifene capsule

| **Ingredient** | **Quantity (mg/capsule)** |
|---|---|
| Raloxifene | 10 |
| Starch, NF | 103 |
| Starch flowable powder | 225.3 |
| Silicone fluid 350 centistokes | 1.7 |

### Formulation 5: Raloxifene capsule

| **Ingredient** | **Quantity (mg/capsule)** |
|---|---|
| Raloxifene | 50 |
| Starch, NF | 150 |
| Starch flowable powder | 397 |
| Silicone fluid 350 centistokes | 3.0 |

The specific formulations above may be changed in compliance with the reasonable variations provided.

A tablet formulation is prepared using the ingredients below:

### Formulation 6: Tablets

| **Ingredient** | **Quantity (mg/tablet)** |
|---|---|
| Active ingredient | 0.1 - 1000 |
| Cellulose, microcrystalline | 0 - 650 |
| Silicon dioxide, fumed | 0 - 650 |
| Stearate acid | 0 - 15 |

The components are blended and compressed to form tablets.

Alternatively, tablets each containing 0.1 -1000 mg of active ingredient are made up as follows:

### Formulation 7: Tablets

| **Ingredient** | **Quantity (mg/tablet)** |
|---|---|
| Active ingredient | 0.1 - 1000 |
| Starch | 45 |
| Cellulose, microcrystalline | 35 |
| Polyvinylpyrrolidone (as 10% solution in water) | 4 |
| Sodium carboxymethyl cellulose | 4.5 |
| Magnesium stearate | 0.5 |
| Talc | 1 |

The active ingredient, starch, and cellulose are passed through a 0.3mm (No. 45 mesh U.S.) sieve and mixed thoroughly. The solution of polyvinylpyrrolidone is mixed with the resultant powders which are then passed through a 1.5mm (No. 14 mesh U.S.) sieve. The granules so produced are dried at 50°-60° C and passed through a 1.0mm (No. 18 mesh U.S.) sieve. The sodium carboxymethyl starch, magnesium stearate, and talc, previously passed through a 0.2mm (No. 60 U.S.) sieve, are then added to the granules which, after mixing, are compressed on a tablet machine to yield tablets.

Suspensions each containing 0.1 - 1000 mg of medicament per 5 mL dose are made as follows:

### Formulation 8: Suspensions

| **Ingredient** | **Quantity (mg/5 ml)** |
|---|---|
| Active ingredient | 0.1 - 1000 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified water to | 5 mL |

The medicament is passed through a 0.3mm (No. 45 mesh U.S.) sieve and mixed with the sodium carboxymethyl cellulose and syrup to form a smooth paste. The benzoic acid solution, flavor, and color are diluted with some of the water and added, with stirring. Sufficient water is then added to produce the required volume.

### TEST PROCEDURES

### Assay 1

The thiobarbituric acid assay as described by Schuh *et al.,* (*PNAS 75*: 3173, 1978) and modified by Morel *et al.,* (*Lab Invest* 55: 419, 1986) was used to determine the degree of inhibition of LDL peroxidation by estradiol and Compound A of the invention. A 1 ml solution containing 250 micrograms (µg) of LDL with either estradiol or Compound A of the invention in amounts varying from 1-30 µM is incubated for 5-18 hrs at 37 degrees C in the presence of 5 µM CuSO₄. Following incubation, 1 ml of 25% trichloroacetic acid and 1 ml of 1% thiobarbituric acid is added. All samples are boiled for 45 minutes and fluorescence is measured at 515 nm excitation and 553 nm emission wavelengths.
(Compound A is a compound of formula 1 wherein R¹ and R³ are hydrogen, and R² is 1-pyrrolidino).

| **TBAR UNITS** | | | | |
|---|---|---|---|---|
| **Conditions** | **25µM** | **5µM** | **1µM** | **0µM** |
| Compound A | 78 | 109 | 142 | 356 |
| Estradiol | 160 | 153 | 259 | 356 |
| Control (no copper) | 90 | 90 | 90 | 90 |

### Assay 2

Resident macrophages from the mouse peritoneal cavity were plated at 1 x 10⁶ cells per well in F10 media without serum. LDL which had been dialized and filtered through a 0.2 micron filter were added to each well at a final concentration of 1 mg/ml (500 µg added per well) and macrophages were treated with 1- 5µM of Compound A or control for 24 hrs. The wells designated media alone represents LDL incubated overnight on the same plate with F10 media without cells. This value was subtracted from the TBAR values obtained in the presence of resident macrophages and reflects the extent of cellular modification of LDL.

| **Conditions** | **TBAR Units** |
|---|---|
| Control | 130 |
| Compound A 5 µM | 30 |
| Compound A 2.5 µM | 75 |
| Compound A 1 µM | 129 |
| Estradiol 25 µM | 30 |

## Claims

1. The use of a compound having the formula 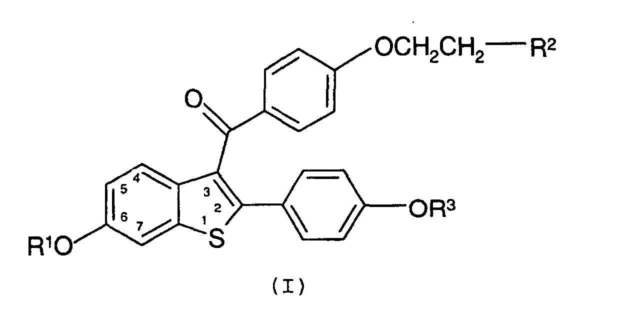 wherein R¹ and R³ are independently hydrogen, -CH₃, 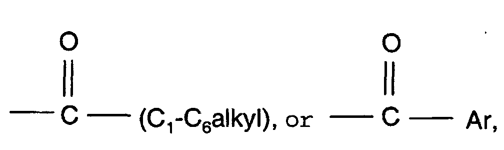 wherein Ar is optionally substituted phenyl;
R² is selected from pyrrolidino and piperidino; or a pharmaceutically acceptable salt or solvate thereof, in the manufacture of a medicament for the treatment of a disorder associated with superoxide anions and other reactive oxygen intermediates.

2. The use of Claim 1 wherein said compound is 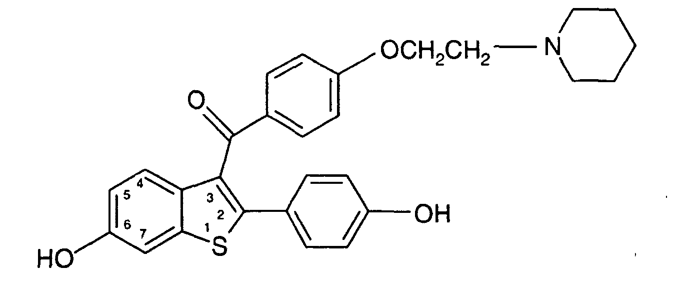 or its hydrochloride salt.

3. The use of Claims 1 or 2 wherein the disorder is Acute Respiratory Distress Syndrome.

4. The use of Claims 1 or 2 wherein the disorder is systemic inflammatory response following cardiopulmonary bypass surgery.

5. The use of Claims 1 or 2 wherein the disorder is pancreatitis.

6. The use of Claims 1 or 2 wherein the disorder is long term respiratory therapy problems associated with high oxygen levels.

## Patentansprüche

1. Verwendung einer Verbindung der Formel 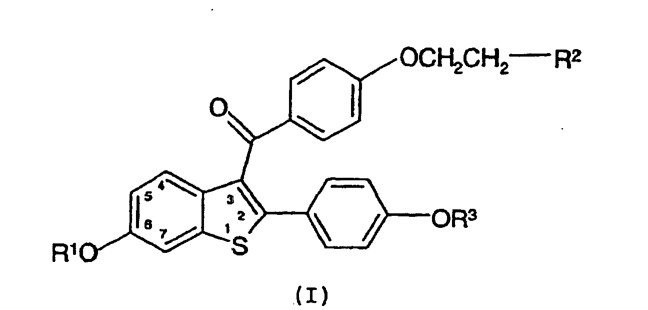 worin
R¹ und R³ unabhängig für Wasserstoff, -CH₃, 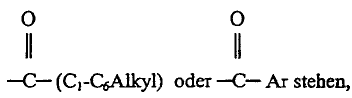 worin Ar für optional substituiertes Phenyl steht, und
R² ausgewählt ist aus Pyrrolidino und Piperidino;
oder eines pharmazeutisch annehmbaren Salzes oder Solvats hiervon,
zur Herstellung eines Arzneimittels für die Behandlung einer Störung, die mit Superoxidanionen und sonstigen reaktiven Sauerstoffzwischenprodukten verbunden ist.

2. Verwendung nach Anspruch 1, worin die Verbindung 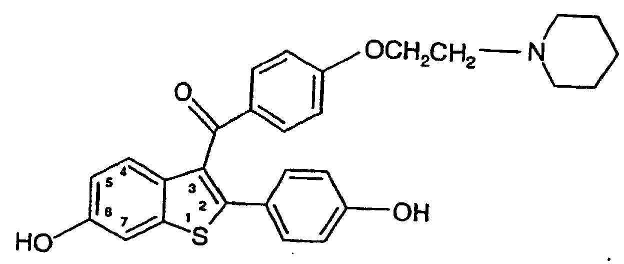 oder ein Hydrochloridsalz hiervon ist.

3. Verwendung nach Anspruch 1 oder 2, worin die Störung akutes Atemnotsyndrom ist

4. Verwendung nach Anspruch 1 oder 2, worin die Störung eine systemische inflammatorische Reaktion nach einer kardiopulmonalen Bypassoperation ist.

5. Verwendung nach Anspruch 1 oder 2, worin die Störung Pankreatitis ist.

6. Verwendung nach Anspruch 1 oder 2, worin die Störung auf mit hohen Sauerstoffspiegeln assoziierten Problemen einer respiratorischen Langzeittherapie beruht.

## Revendications

1. Utilisation d'un composé répondant à la formule 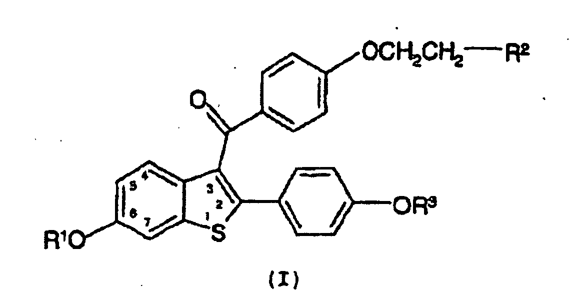 dans laquelle R¹ et R³ représentent, indépendamment l'un de l'autre, un atome d'hydrogène,
un groupe -CH₃, 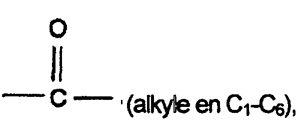 ou 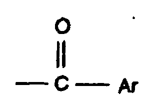 dans lequel Ar représente un groupe phényle facultativement substitué ;
R² est choisi parmi les groupes pyrrolidino et pipéridino ; ou un sel ou solvate pharmaceutiquement acceptable de celui-ci, dans la fabrication d'un médicament destiné au traitement d'un trouble associé à des anions superoxydes et à d'autres intermédiaires oxygénés réactifs.

2. Utilisation du composé selon la revendication 1 dans lequel ledit composé répond à la formule 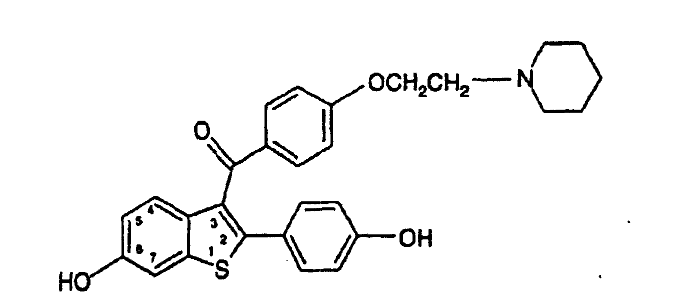 ou représente son sel d'hydrochlorure.

3. Utilisation selon la revendication 1 ou 2 dans lequel le trouble est le Syndrome de Détresse Respiratoire Aigu.

4. Utilisation selon la revendication 1 ou 2 dans lequel le trouble est une réponse inflammatoire systémique consécutive à une intervention chirurgicale de pontage cardio-pulmonaire.

5. Utilisation selon la revendication 1 ou 2 dans lequel le trouble est une pancréatite.

6. Utilisation selon la revendication 1 ou 2 dans lequel le trouble consiste en des problèmes de thérapie respiratoire de longue durée associés à des niveaux élevés d'oxygène.
